(19) **Europäisches Patentamt / European Patent Office / Office européen des brevets**

(11) **EP 3 882 630 A1**

(12) **EUROPEAN PATENT APPLICATION**
published in accordance with Art. 153(4) EPC

(43) Date of publication:
**22.09.2021 Bulletin 2021/38**

(21) Application number: **21712937.8**

(22) Date of filing: **02.02.2021**

(51) Int Cl.:
**G01N 33/53** (2006.01) **G01N 33/543** (2006.01)

(86) International application number:
**PCT/JP2021/003773**

(87) International publication number:
**WO 2021/157576 (12.08.2021 Gazette 2021/32)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **06.02.2020 JP 2020018653**

(71) Applicant: **National University Corporation Okayama University**
**Kita-ku**
**Okayama-shi**
**Okayama 700-8530 (JP)**

(72) Inventors:
- **MATSUURA, Eiji**
  **Okayama 7008530 (JP)**
- **TAKENAKA, Fumiaki**
  **Okayama 7008530 (JP)**
- **TAN, Xian Wen**
  **Okayama 7008530 (JP)**

(74) Representative: **Daniels, Jeffrey Nicholas**
**Page White & Farrer**
**Bedford House**
**John Street**
**London WC1N 2BF (GB)**

(54) **METHOD FOR DETECTING OXIDIZED LDL/BETA2GPI COMPLEX, AND DETECTION KIT**

(57) The object of the present invention is to provide an easy and quick detection method for detecting an oxLDL/$\beta_2$GPI complex in biological samples, and a detection kit therefor. The present invention attains this object by providing a detection method for detecting an oxLDL/$\beta_2$GPI complex, which uses a test strip for lateral flow assay, comprising a step of capturing the oxLDL/$\beta_2$GPI complex in the test sample in a predetermined position on the test strip by a first binding component that binds to the oxLDL/$\beta_2$GPI complex; and a step of labeling the oxLDL/$\beta_2$GPI complex captured in the predetermined position on the test strip by making a second binding component comprising a labeling agent be bound to the captured oxLDL/$\beta_2$GPI complex, and a detection kit therefor.

Figure 5

Sequence Listing

OP01414 Sequence Listing.txt

SEQUENCE LISTING

<110> NATIONAL UNIVERSITY CORPORATION OKAYAMA UNIVERSITY

<120> A method for measuring oxidized LDL/beta2GPI complex and a kit therefor

<130> OP01414-PCT

<160> 8

<210> 1
<211> 118
<212> PRT
<213> Mus musculus

<400> 1

```
Glu Val Gln Leu Val Glu Ser Gly Gly Gly Leu Val Lys Pro Gly Gly
1               5                   10                  15
Ser Leu Lys Leu Ser Cys Ala Ala Ser Gly Phe Thr Phe Ser Ser Tyr
            20                  25                  30
Ala Met Ser Trp Ala Arg Gln Thr Pro Glu Lys Arg Leu Glu Trp Val
        35                  40                  45
Ala Thr Ile Ser Ser Gly Gly Ser Tyr Thr Tyr Tyr Pro Asp Ser Val
    50                  55                  60
Arg Gly Arg Phe Thr Ile Ser Arg Asp Asn Ala Lys Asn Thr Leu Tyr
65                  70                  75                  80
Leu Gln Met Cys Ser Leu Arg Ser Glu Asp Thr Ala Met Tyr Tyr Cys
                85                  90                  95
Ala Arg Phe Asp Gly Tyr Tyr Ala Met Asp Tyr Trp Gly Gln Gly Thr
            100                 105                 110
Ser Val Thr Val Ser Ser
            115
```

<210> 2
<211> 5
<212> PRT
<213> Mus musculus

<400> 2

```
Ser Tyr Ala Met Ser
1               5
```

<210> 3
<211> 17
<212> PRT
<213> Mus musculus

<400> 3

```
Thr Ile Ser Ser Gly Gly Ser Tyr Thr Tyr Tyr Pro Asp Ser Val Arg
1               5                   10                  15
Gly
```

<210> 4
<211> 9
<212> PRT
<213> Mus musculus

<400> 4

```
Phe Asp Gly Tyr Tyr Ala Met Asp Tyr
```

1                     5

<210>  5
<211>  107
<212>  PRT
<213>  Mus musculus

<400>  5

Asp Ile Val Met Thr Gln Ser His Lys Phe Met Ser Thr Ser Val Gly
1               5                   10                  15
Asp Arg Val Ser Ile Thr Cys Lys Ala Ser Gln Asp Val Gly Ser Ala
                20                  25                  30
Val Ala Trp Tyr Gln Gln Lys Pro Gly Gln Ser Pro Lys Leu Leu Ile
                35                  40                  45
Tyr Trp Ala Ser Thr Arg His Thr Gly Val Pro Asp Arg Phe Thr Gly
        50                  55                  60
Ser Gly Ser Gly Thr Asp Phe Thr Leu Thr Ile Ser Ser Leu Gln Ser
65                  70                  75                  80
Glu Asp Leu Ala Asp Tyr Phe Cys Gln Gln Tyr Ser Ser Tyr Pro Leu
                85                  90                  95
Thr Phe Gly Ser Gly Thr Lys Leu Glu Ile Lys
            100                 105

<210>  6
<211>  11
<212>  PRT
<213>  Mus musculus

<400>  6

Lys Ala Ser Gln Asp Val Gly Ser Ala Val Ala
1               5                   10

<210>  7
<211>  7
<212>  PRT
<213>  Mus musculus

<400>  7

Trp Ala Ser Thr Arg His Thr
1               5

<210>  8
<211>  9
<212>  PRT
<213>  Mus musculus

<400>  8

Gln Gln Tyr Ser Ser Tyr Pro Leu Thr
1               5

## Description

<u>Technical Field</u>

The present invention relates to a detection method for detecting an oxLDL/$\beta_2$GPI complex in a test sample, and a detection kit.

<u>Background Art</u>

In a variety of arteriosclerotic diseases including antiphospholipid syndrome (APS), it has been revealed that oxLDL/$\beta_2$GPI complex is present in blood of patients and involves progression of arteriosclerosis. By measuring the amount of oxLDL/$\beta_2$GPI complex in blood, the size of arteriosclerotic lesion can be estimated and thereby progression of arteriosclerosis can be monitored.

As a method for detecting or quantifying an oxLDL/$\beta_2$GPI complex, ELISA (Enzyme-Linked Immuno Sorbent Assay) method is known (Patent Literature 1). However, ELISA method requires a staff with high experimental skill for conducting examination and is not suitable for high-throughput analysis, because ELISA involves repetition of the laborious experimental procedure including addition of a variety of reagents, incubation, and washing.

<u>Prior Art Literature</u>

<u>Patent Literature</u>

Patent Literature 1: Japanese Patent No. 5616592

<u>Disclosure of Invention</u>

<u>Object of the Invention</u>

The present invention was made to solve problems in the above prior arts. An object of the present invention is to provide an easy and quick detection method for detecting an oxLDL/$\beta_2$GPI complex in a biological sample and a detection kit used for such a detection method.

<u>Means to Attain the Object</u>

The present inventors eagerly made a continuous research effort in order to attain the above object, and found that a measurement system using lateral flow enables much quicker and easier detection and quantification of an oxLDL/$\beta_2$GPI complex in a test sample, surprisingly, with broader dynamic range than the conventional ELISA method.

The present invention attains the above-mentioned object by providing a detection method for detecting a complex of oxidized LDL and $\beta_2$-glycoprotein I (an oxLDL/$\beta_2$GPI complex) in a test sample,

which uses a test strip for lateral flow assay and comprises;
a step of capturing the oxLDL/$\beta_2$GPI complex in the test sample in a predetermined position on the test strip by a first binding component that binds to the oxLDL/$\beta_2$GPI complex; and
a step of labeling the oxLDL/$\beta_2$GPI complex captured in the predetermined position on the test strip by making a second binding component comprising a labeling agent be bound to the captured oxLDL/$\beta_2$GPI complex.

As the first binding component that binds to the oxLDL/$\beta_2$GPI complex, the first binding component that binds to any part of the oxLDL/$\beta_2$GPI complex under any mechanism may be used as long as the first binding component is able to bind to the oxLDL/$\beta_2$GPI complex and the oxLDL/$\beta_2$GPI complex can be captured in a predetermined position on a test strip for lateral flow assay. However, according to findings of the present inventors, it is preferable that the first binding component comprises a first binding member that specifically binds to a $\beta_2$GPI comprised in an oxLDL/$\beta_2$GPI complex. As such a first binding component, anti-$\beta_2$GPI antibody that specifically binds to a $\beta_2$GPI comprised in an oxLDL/$\beta_2$GPI complex may be preferably used. By using anti-$\beta_2$GPI antibody as the first binding component, an oxLDL/$\beta_2$GPI complex in a test sample can be detected and measured with high sensitivity.

The reason why an oxLDL/$\beta_2$GPI complex in a test sample can be detected with high sensitivity by using the first binding component comprising the first binding member that specifically binds to a $\beta_2$GPI comprised in the oxLDL/$\beta_2$GPI complex is presumed as below. In the detection method of the present invention, the first binding component plays a role of binding to an oxLDL/$\beta_2$GPI complex and capturing the oxLDL/$\beta_2$GPI complex in a predetermined position on a test strip. Meanwhile, one oxLDL/$\beta_2$GPI complex, which is a target of detection, comprises one LDL particle and usually more than two $\beta_2$GPI molecules, depending on the status of disease, as constituents (Journal of Lipid Research 2001, vol. 42, no. 5, pp. 697-709.). When the first binding component comprising the first binding member that specifically binds to a $\beta_2$GPI molecule comprised in an oxLDL/$\beta_2$GPI complex, the first binding component is able to bind to an oxLDL/$\beta_2$GPI complex at multiple binding sites, and accordingly an oxLDL/$\beta_2$GPI complex can be captured in a predetermined position on a test strip with higher probability. Examples of a first binding component that specifically binds to a $\beta_2$GPI molecule comprised in an oxLDL/$\beta_2$GPI complex, include anti-$\beta_2$GPI antibody as mentioned above.

In a preferred embodiment, the present invention is directed to the detection method, wherein the first binding component comprises a first binding unit, which comprises a first specific binding element and the first binding member that specifically binds to a $\beta_2$GPI molecule comprised in the oxLDL/$\beta_2$GPI complex, and a second binding unit, which is placed in the predetermined position on the test strip and comprises a second specific binding element that specifically binds to the first specific binding element, and
wherein the step of capturing the oxLDL/$\beta_2$GPI complex in the test sample in the predetermined position on the test strip comprises, a step of making the oxLDL/$\beta_2$GPI complex and the first binding member comprised in the first binding unit be bound, and a step of making the first specific binding element of the first binding unit and the second specific binding element of the second binding unit be bound. The step of making the oxLDL/$\beta_2$GPI complex and the first binding member comprised in the first binding unit be bound may be performed on the test strip or outside the test strip.

When the first binding component comprises the first binding unit and the second binding unit, the step of capturing the oxLDL/$\beta_2$GPI complex in the predetermined position on the test strip by the first binding component is performed in two separate steps, namely a step of making the oxLDL/$\beta_2$GPI complex and the first binding member comprised in the first binding unit be bound, and a step of making the first specific binding element comprised in the first binding unit and the second specific binding element comprised in the second binding unit be bound. As a result, in terms of the first binding member of the first binding unit for example, it is advantageous in that the first binding member that specifically binds to the oxLDL/$\beta_2$GPI complex can be selected, with placing more importance on the specificity of binding to the oxLDL/$\beta_2$GPI complex rather than the easiness for fixing it temporally on a test strip. On the other hand, in terms of the first specific binding element of the first binding member and the second specific binding element of the second binding member, the combination of the first and the second specific binding element can be selected, with placing more importance on the capturing efficiency of a detection target that comes transported by lateral flow rather than the specificity of binding to the oxLDL/$\beta_2$GPI complex, which is advantageous in that the detection with higher sensitivity is enabled

The step of making the oxLDL/$\beta_2$GPI complex and the first binding member comprised in the first binding unit be bound, and the step of making the first specific binding element comprised in the first binding unit and the second specific binding element comprised in the second binding unit be bound may be performed simultaneously or separately in any order. However, it is preferable to perform the step of making the oxLDL/$\beta_2$GPI complex and the first binding member comprised in the first binding unit be bound before performing the step of making the first specific binding element comprised in the first binding unit and the second specific binding element comprised in the second binding unit be bound, and at the upper stream of lateral flow if the step is performed on a test strip. Although the step of making the oxLDL/$\beta_2$GPI complex and the first binding member comprised in the first binding unit be bound may be performed on a test strip, it may be preferable to perform the step outside a test strip. If the step is performed outside a test strip, it is advantageous in that a condition such as reaction time and temperature appropriate for making the oxLDL/$\beta_2$GPI complex and the first binding member comprised in the first binding unit be bound may be selected with a high degree of freedom.

As the first specific binding element and the second specific binding element comprised in the first binding unit and the second binding unit, respectively, any pair of substances forming a specific and strong binding may be used. In a preferred embodiment of the detection method of the present invention, the first specific binding element and the second specific binding element may be avidin and biotin or *vice versa,* streptavidin and biotin or *vice versa,* or neutravidin and biotin or *vice versa,* respectively. These pairs of specific binding elements form very strong bonding whose dissociation constant is as low as $10^{-15}$ M. This binding is stronger than the binding formed between oxLDL/$\beta_2$GPI complex and antibody, antibody fragment, aptamer, and peptide, which are typically used as a substance that provides a binding member comprised in the first binding component in the detection method of the present invention, providing an advantage that a complex of the first binding unit and an oxLDL/$\beta_2$GPI complex that comes transported by lateral flow can be efficiently and fully captured in the predetermined position on the test strip and accordingly the detection sensitivity is enhanced. Herein, "avidin and biotin or *vice versa"* means that avidin and biotin may be used as the first specific binding element and the second specific binding element, respectively, or, alternatively, avidin and biotin may be used as the second specific binding element and the first specific binding element, respectively. The same applies to remaining "streptavidin and biotin or *vice versa"* and "neutravidin and biotin or *vice versa".*

Meanwhile, in a preferred embodiment of the present invention, the second binding component comprises a second binding member that binds to an apolipoproteinB-100 (apoB100) comprised in the oxLDL/$\beta_2$GPI complex.

One oxLDL/$\beta_2$GPI complex, a detection target of the detection method of the present invention, comprises one oxLDL particle per complex. One oxLDL particle usually comprises one apoB100 per particle (Journal of Lipid Research 2001, vol. 42, no. 9, pp. 1346-1367; Biological and Pharmaceutical Bulletin 2016, vol. 39, no. 1, pp. 1-24). Therefore, labeling the oxLDL/$\beta_2$GPI complex by the second binding component comprising a binding member that binds to an apoB100 would result in binding of one labeling agent per oxLDL/$\beta_2$GPI complex, which is being captured in the predetermined position on the test strip. Thus, a highly quantitative detection of an oxLDL/$\beta_2$GPI complex may be enabled.

In a preferred embodiment, the present invention provides the detection method for detecting an oxLDL/$\beta_2$GPI complex, wherein binding between the first binding member and the oxLDL/$\beta_2$GPI complex is based on antigen-antibody binding, and the detection method for detecting an oxLDL/$\beta_2$GPI complex, wherein binding between the second binding member and the oxLDL/$\beta_2$GPI complex is based on antigen-antibody binding.

As a labeling agent comprised in the second binding components, any labeling agent that is used in the art may be used as long as it functions as labeling agent and the presence or absence of labeling agent and furthermore the quantity of labeling agents can be detected. In a preferred embodiment, the labeling agent may be metal nanoparticles including gold nanoparticles, platinum nanoparticles, silver nanoparticles, and gold-platinum alloy nanoparticles, or colloidal quantum dots.

On the other hand, the present invention attains the above-mentioned object further by providing a detection kit for carrying out above-described detection methods.

Effect of the Invention

In accordance with a detection method for detecting an oxLDL/$\beta_2$GPI complex of the present invention, an oxLDL/$\beta_2$GPI complex contained in a test sample including a biological sample can be detected or quantified more easily and quickly with broader dynamic range when compared to a conventional ELISA method. Meanwhile, in accordance with a detection kit of the present invention, an oxLDL/$\beta_2$GPI complex contained in a test sample can be easily and quickly detected or quantified without necessitating professional skill.

Brief Description of Drawings

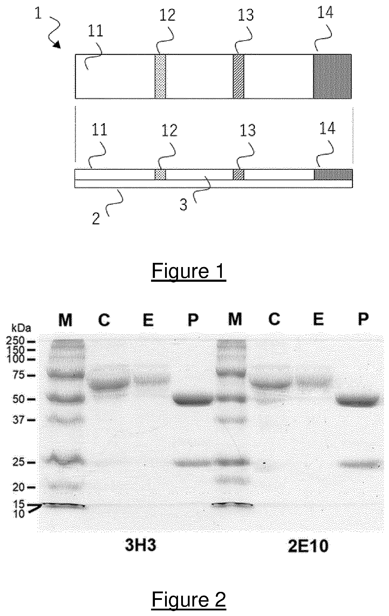

Figure 1. A schematic illustration of an example of a test strip for lateral flow assay

Figure 2. A photograph showing the result of SDS-PAGE. Each lane corresponds to the following samples: Protein molecular weight standard (M), cell culture supernatant of hybridoma cells producing 3H3 antibody or 2E10 antibody (C), effluent obtained during purification of 3H3 or 2E10 antibody (E), and purified 3H3 or 2E10 antibody (P). Samples were reduced by 2-mercaptomethanol before being subjected to electrophoresis.

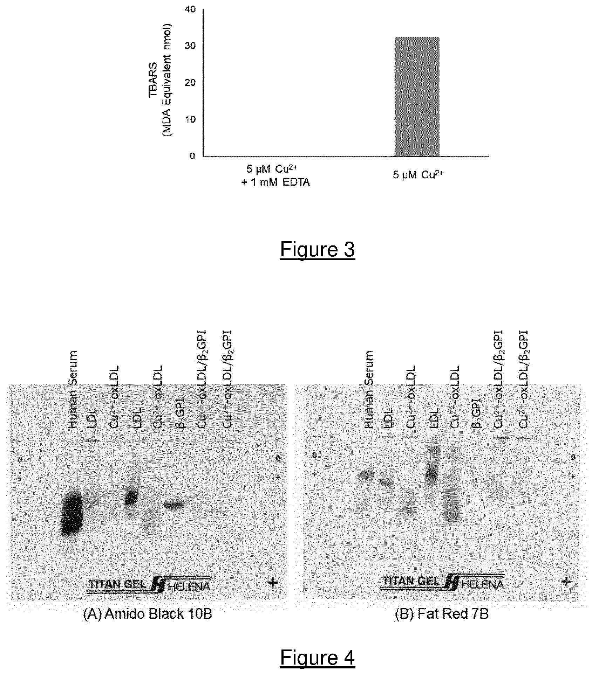

Figure 3. A graph showing the result of TBARS assay. The amount of TBARS (Thiobarbituric Reactive Substance) produced in the LDL sample, where the oxidation was induced using copper (II) sulfate, and the comparative sample, where the oxidation by copper (II) sulfate was inhibited by addition of ethylenediaminetetraacetic acid, was detected. The amount of TBARS was expressed as nmol MDA equivalent.

Figure 4. A photograph showing the result of the agarose gel electrophoresis. In the figure, (A) shows the result when protein is stained using Amido Black 10B, and (B) shows that result when lipid is stained using Fat Red 7B.

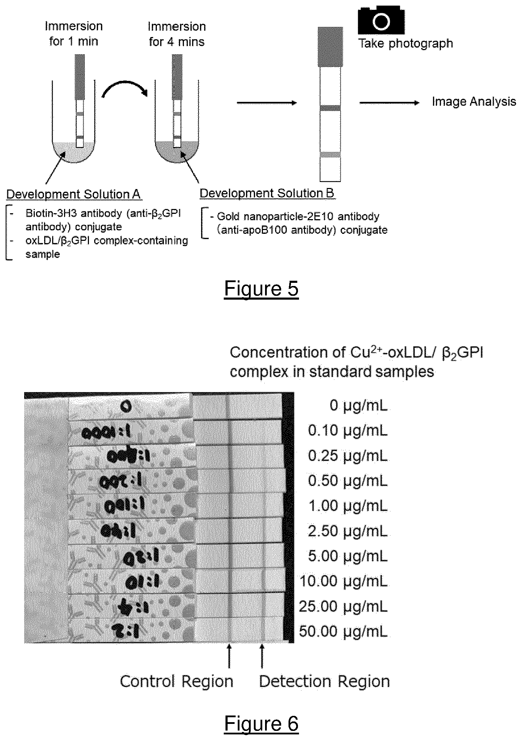

Figure 5. A schematic illustration of the experimental procedure of an example of the detection method of the present invention.

Figure 6. A photograph of test strips after flow of development solution A and development solution B obtained in Experiment 5. As test sample, standard samples containing known concentrations of $Cu^{2+}$-oxLDL/$\beta_2$GPI complex were used.

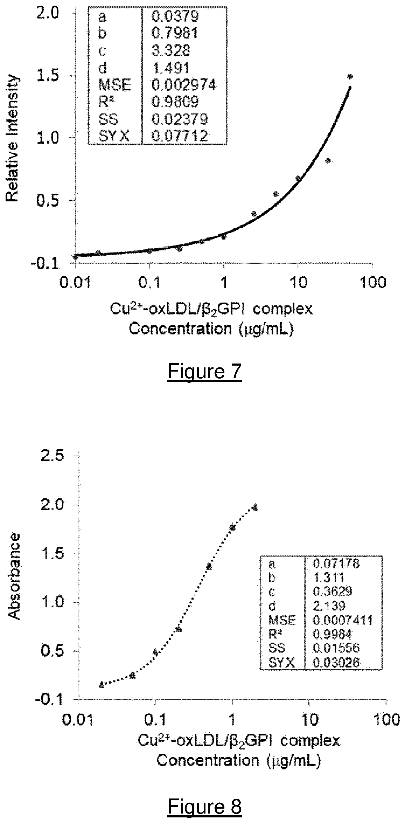

Figure 7. A graph showing a standard curve obtained with an example of the detection method of the present invention (Experiment 5). Standard samples containing known concentrations of $Cu^{2+}$-oxLDL/$\beta$2GPI complex was used as sample, and measurement data were obtained by the detection method of the present invention. The obtained measurement data were plotted and a standard curve was obtained by applying four parametric logistic (4PL) regression model.

Figure 8. A graph showing a standard curve obtained with an ELISA method (Experiment 6). Standard samples containing known concentrations of $Cu^{2+}$-oxLDL/β2GPI complex was used as sample, and measurement data were obtained by the detection method of the present invention. The obtained measurement data were plotted and a standard curve was obtained by applying four parametric logistic (4PL) regression model.

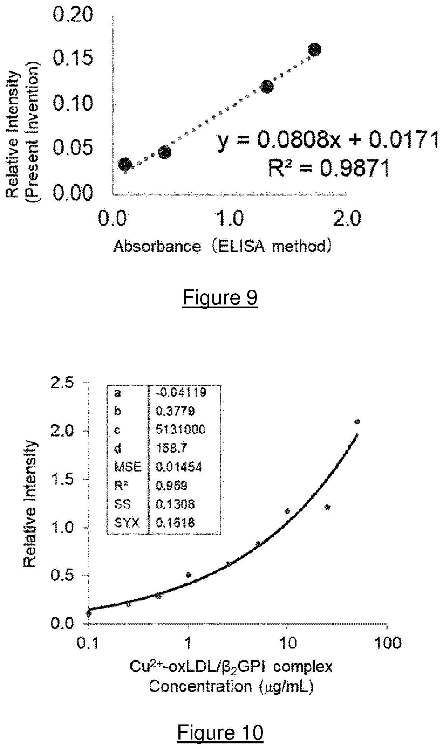

Figure 9. A correlation diagram showing a relationship between the measurement data (relative intensity) obtained from standard samples with an example of the detection method of the present invention and the measurement data (absorbance) obtained from the same standard samples with an ELISA method.

Figure 10. A graph showing a standard curve obtained with an example of the detection method of the present invention (Experiment 7). Standard samples containing known concentrations of $Cu^{2+}$-oxLDL/β2GPI complex was used as sample, and measurement data were obtained by the detection method of the present invention. The obtained measurement data were plotted and a standard curve was obtained by applying four parametric logistic (4PL) regression model.

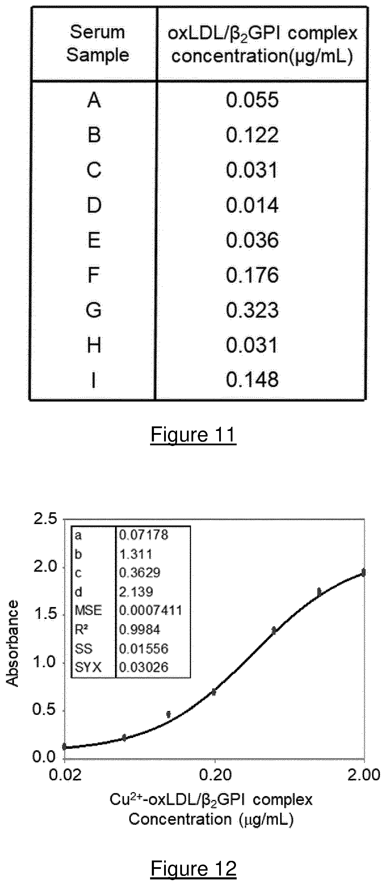

Figure 11. A figure showing the concentration of oxLDL/$β_2$GPI complex in serum samples A to I determined by an example of the detection method of the present invention. By using the standard curve shown in Figure 10, the concentration of oxLDL/$β_2$GPI complex in each serum sample was semi-quantitatively determined.

Figure 12. A graph showing a standard curve obtained with an ELISA method (Experiment 7). Standard samples containing known concentrations of $Cu^{2+}$-oxLDL/β2GPI complex was used as sample, and measurement data were obtained by the detection method of the present invention. The obtained measurement data were plotted and a standard curve was obtained by applying four parametric logistic (4PL) regression model.

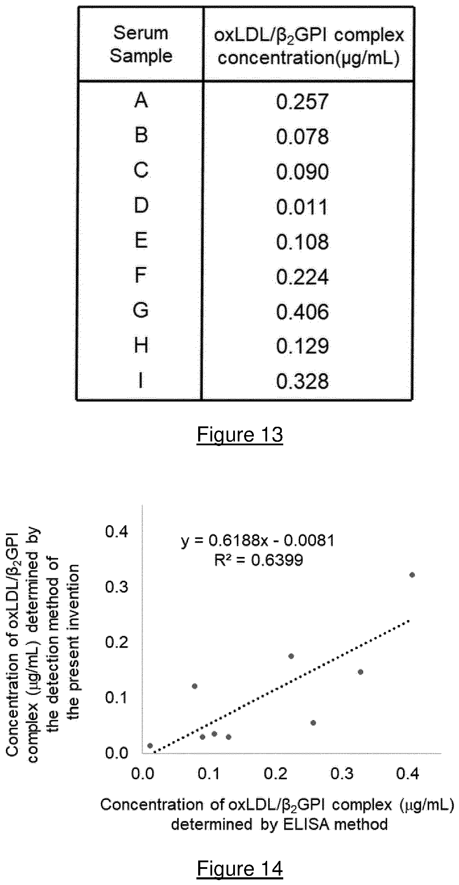

Figure 13. Afigure showing the concentration of oxLDL/$β_2$GPI complex in serum samples A to I determined by an ELISA method. By using the standard curve shown in Figure 12, the concentration of oxLDL/$β_2$GPI complex in each serum sample was semi-quantitatively determined.

Figure 14. A correlation diagram showing a relationship between the concentration of oxLDL/$β_2$GPI complex in serum samples A to I determined by an example of the detection method of the present invention and the concentration of oxLDL/$β_2$GPI complex in serum samples Ato I determined by an ELISA method.

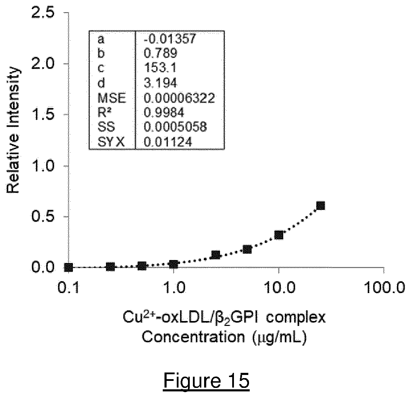

Figure 15. A graph showing a standard curve obtained with an example of the detection method of the present invention (Experiment 8). Standard samples containing known concentrations of $Cu^{2+}$-oxLDL/β2GPI complex was used as sample, and measurement data were obtained by the detection method of the present invention. The obtained measurement data were plotted and a standard curve was obtained by applying four parametric logistic (4PL) regression model.

Mode for Carrying Out the Invention

<1. Explanation of Terms>

<1.1. Test Sample>

First, a "test sample" that is applied to the detection method of the present invention, may be any test sample as long as there is a possibility that the test sample may contain an oxLDL/$β_2$GPI complex. Typical examples of a test sample include, but not limited to, any specimens which are taken from such as patients of arteriosclerotic diseases, in particular, blood specimens including serum, plasma, and whole blood. These test samples may be directly used for the detection or may as well be diluted with appropriate diluent before the detection. Test samples may as well be filtered before the detection.

<1.2. Detect>

In the pr the amount of the substance quantitatively or sem i-quantitatively. As described later, in the detection method of the present invention, the amount of oxLDL/$β_2$GPI complex contained in a variety of test samples can be quantitatively or semi-quantitatively obtained by fitting measurement data obtained using test samples to standard curves obtained using standard samples containing known concentrations of standard substance.
esent description, the term "detect" means not only to determine the presence or absence of a substance that is a detection target, but also to measure

<1.3. Test Strip for Lateral Flow Assay>

The term "Test Strip for Lateral Flow Assay" means a test strip that is used when detecting or quantifying measurement target in test samples using lateral flow method. The following is an explanation for "Test Strip for Lateral Flow Assay" (hereinafter also referred to simply as "test strip") used for the detection method of the present invention. Figure 1 is a schematic illustration of an example of a test strip for lateral flow assay used in the detection method of the present invention. In the upper part of Figure 1, plan view of the example of a test strip is provided, while in the lower part of Figure 1, vertical section of the same example of a test strip is provided. In Figure 1, numeral 1 indicates a test strip for lateral flow assay. Test strip for lateral flow assay 1 is a test strip for detecting an oxLDL/$\beta_2$GPI complex contained in a test sample that will be an object of measurement, and consists of supporting member 2 and membrane 3, which is positioned on supporting member 2 and integrated to supporting member 2, as shown in Figure 1. Membrane 3 has sample pad 11, detection region 12, control region 13, and wicking pad 14 in this order from upstream (in Figure 1, left side) of the flow of a test sample to downstream of the flow, wherein sample pad 11 is a part at which test strip 1 comes into contact with a test sample and takes a test sample into test strip 1, detection region 12 is a part at which an oxLDL/$\beta_2$GPI complex in a test sample is captured, control region 13 is a part at which a second binding component is captured, and wicking pad 14 is a part which absorbs liquid that flows through a test strip and reaches wicking pad 14. The size of test strip 1 is not particularly limited, however, for example, a test strip having a width of 3 mm to 10 mm and a length between sample pad 11 and wicking pad 14 of 5 mm to 30 mm can be appropriately used.

<1.3.1. Detection Region>

Detection region 12 is a part at which an oxLDL/$\beta$2GPI complex in a test sample, which is taken into membrane 3 of test strip 1 through sample pad 11, is captured. In the detection method of the present invention, an oxLDL/$\beta_2$GPI complex in a test sample is captured in a predetermined position on a test strip, namely, detection region 12, by a first binding component that binds to the complex. A second binding component comprising a labeling agent then binds to the oxLDL/$\beta_2$GPI complex thus captured in detection region 12, thereby the oxLDL/$\beta$2GPI complex is labeled by the labeling agent. The amount of the second binding component, in other words the amount of the labeling agent, bound to the complex captured in detection region 12 depends on the amount of the oxLDL/$\beta$2GPI complex captured in detection region 12. Accordingly, by detecting or quantifying the labeling agent captured in detection region 12, oxLDL/$\beta_2$GPI complex in the test sample can be detected or quantified.

<1.3.2. Control Region>

In a preferred embodiment, test strip 1 used for the detection method of the present invention comprises control region 13. Control region 13, positioned at downstream of detection region 12, is a part which gets in contact with liquid that passes through detection region 12 and which comprises a substance that binds to a second binding component. When control region 13 is provided, a second binding component that passes through detection region 12 without being bound to the oxLDL/$\beta_2$GPI complex captured in detection region 12 can be captured in control region 13. As mentioned above, a second binding component comprises a labeling agent. Accordingly, the successful transport and movement of a test sample by lateral flow in membrane 3 can be confirmed by detecting the presence or absence of the labeling agent captured in control region 13.

<1.3.3. Sample Pad>

Sample pad 11 is a part for taking in a test sample inside test strip 1. A test sample is taken into membrane 3 of test strip 1 when sample pad 11 gets in contact with a liquid test sample. The contact of sample pad 11 and a test sample can be performed, for example, by dropping a liquid test sample onto sample pad 11 or by immersing sample pad 11 in a liquid test sample. Sample pad 11 may be placed anywhere of a test strip as long as a test sample can be transported through membrane 3 of test strip 1 by capillary-flow and reach detection region 12, control region 13, and wicking pad 14. However, in terms of easiness for making it in contact with a test sample, it is preferable to place sample pad 11 at an edge of test strip 1.

<1.3.4. Wicking Pad>

Wicking pad 14 is a part that absorbs liquid that comes transported through membrane 3 of test strip 1 by lateral flow and promotes transport of a liquid test sample through a test strip by capillary flow. Wicking pad 14 is composed of materials with absorbability.

<1.3.5. Supporting Member>

In a preferred embodiment, membrane 3 provided with sample pad 11, detection region 12, control region 13, and wicking pad 14 composing test strip 1 is placed on supporting member 2. Supporting member 2 may be composed of any material as long as it is able to support membrane 3 and does not obstruct capillary flow in membrane 3. Such materials include, for example but not limited to, plastic, glass, ceramic, and paper. Membrane 3 provided with sample pad 11, detection region 12, control region 13, and wicking pad 14 may be adhered to supporting member 2 using an appropriate adhesive. Any type of adhesive may be used as long as it does not obstruct capillary flow in membrane 3.

<1.3.6. Membrane Materials>

Membrane 3 can be composed on any type of material as long as a test sample can be absorbed to membrane 3 and transported by capillary flow. Preferred examples of materials composing membrane 3 include, for example but not limited to, cellulose and cellulose derivatives such as cellulose acetate, and nitrocellulose, polyester, polyethylene, polyethersulfone, polycarbonate, nylon, acrylic fiber, and glass fiber. Meanwhile, membrane 3 composing test strip 1 may contain a blocking agent such as bovine serum albumin (BSA), casein, and sucrose to reduce unspecific adsorption of proteins and so on to membrane 3.

<1.4. First Binding Component>

A first binding component is a binding component that binds to an oxLDL/$\beta_2$GPI complex in a test sample and plays a role in capturing the oxLDL/$\beta_2$GPI complex in a predetermined position on test strip 1, namely detection region 12. A first binding component may consist of any substance or combination of substances as long as it is able to bind to an oxLDL/$\beta_2$GPI complex and is able to have the thus bound oxLDL/$\beta_2$GPI complex captured in detection region 12. A first binding component may bind to any part of an oxLDL/$\beta_2$GPI complex. Examples of a first binding component include any antibody, antibody fragment, peptide, DNA aptamer, RNA aptamer, modified product thereof, and complex thereof.

In a preferred embodiment, a first binding component may comprise a first binding member that specifically binds to a $\beta_2$GPI comprised in an oxLDL/$\beta_2$GPI complex, in terms of achieving detection with high sensitivity. Specifically binding to a $\beta_2$GPI comprised in an oxLDL/$\beta_2$GPI complex means that a first binding member binds more strongly to a $\beta_2$GPI comprised in an oxLDL/$\beta_2$GPI complex than to a $\beta_2$GPI that is not bound to an oxLDL. Examples of a substance that features such a binding member include, but not limited to, the following antibodies (a) to (e) disclosed in Japanese Patent No. 5616592:

(a) an antibody comprising a heavy chain that comprises CDR1 having the amino acid sequence of SEQ ID NO: 2, CDR2 having the amino acid sequence of SEQ ID NO: 3, and CDR3 having the amino acid sequence of SEQ ID NO: 4;
(b) an antibody comprising a heavy chain that comprises a heavy-chain variable region having the amino acid sequence of SEQ ID NO: 1;
(c) an antibody comprising a light chain that comprises CDR1 having the amino acid sequence of SEQ ID NO: 6, CDR2 having the amino acid sequence of SEQ ID NO: 7, and CDR3 having the amino acid sequence of SEQ ID NO: 8;
(d) an antibody comprising a light chain that comprises a light-chain variable region having the amino acid sequence of SEQ ID NO: 5; and
(e) an antibody that comprises a pair of the heavy chain of (a) or (b) above and the light chain of (c) or (d) above.

A more specific example includes an anti-$\beta_2$GPI antibody produced by hybridoma clone 3H3 established by a cell fusion method, which is disclosed also in Japanese Patent No. 5616592. Meanwhile, the above antibodies can be prepared by the method disclosed in Japanese Patent No. 5616592.

As described above, an oxLDL/$\beta_2$GPI complex in a test sample can be detected with high sensitivity when a first binding component comprising a first binding member that specifically binds to a $\beta_2$GPI comprised in an oxLDL/$\beta_2$GPI complex is used. The reason why a high sensitivity can be achieved is still not fully revealed, however, the reason may be presumed as below. As already described, one oxLDL/$\beta_2$GPI complex contains one oxLDL particle and usually two or more $\beta_2$GPI molecules as constituents, depending on the status of diseases. Accordingly, when a first binding component comprises a first binding member that specifically binds to a $\beta_2$GPI molecule comprised in an oxLDL/$\beta_2$GPI complex, an oxLDL/$\beta_2$GPI complex can be captured in a predetermined position of a test strip in a higher probability because the first binding component is capable of binding to an oxLDL/$\beta_2$GPI complex at multiple binding sites.

<1.5. Second Binding Component>

Meanwhile, a second binding component is a binding component comprising a labeling agent, which binds to and thereby labels the oxLDL/$\beta_2$GPI complex captured in detection region 12 by a first binding component, so that it can be detected with an appropriate method. Any type of substance or combination of substances may be used as a second binding component as long as it is able to bind to an oxLDL/$\beta_2$GPI complex. Examples of a second binding component include, but not limited to, any antibody, antibody fragments, peptides, DNA aptamer, RNA aptamer, modified product thereof, and complexes thereof.

Meanwhile, in terms of achieving better sensitivity of detection, it is preferable that a second binding component comprises a second binding member that specifically binds to an apoB100 comprised in an oxLDL/$\beta_2$GPI complex. As already mentioned, one oxLDL/$\beta_2$GPI complex, which is a detection target of the detection method of the present invention, usually comprises one apoB100 per complex. If a second binding component comprises a second binding member that binds to an apoB100, each one of oxLDL/$\beta_2$GPI complexes captured in detection region 12 will be bound by one labeling agent, enabling more quantitative detection of an oxLDL/$\beta_2$GPI complex. Examples of a substance that comprises such a second binding member include anti-apoB100 antibody. As an anti-apoB100 antibody, for example, N2E10 antibody that is disclosed in Japanese Patent Kokai No. 2005-097203 may be used.

<1.6 Labeling Agent>

As a labeling agent comprised in a second binding component, any labeling agent that is being used in the art can be used as long as it works as labeling agent and the presence or absence and further the amount of the agent can be detected. Examples of such a labeling agent that may be used in the detection method of the present invention include, but not limited to, metal nanoparticles such as gold nanoparticles, platinum nanoparticles, silver nanoparticles, and gold-platinum alloy nanoparticles, colloidal quantum dots (QDs), polymer particles loaded with dyes such as colored latex beads, silica nanoparticles loaded with dyes, and carbon nanoparticles. In terms of easiness of modification on binding components, sensitivity and accuracy of detection, as well as easiness of detection, metal nanoparticles such as gold nanoparticles, platinum nanoparticles, silver nanoparticles, and gold-platinum alloy nanoparticles may be preferably used as labeling agent, and more preferably gold nanoparticles are used as labeling agent. In particular, gold nanoparticles with an average diameter of approximately 5 to 250 nm are preferable and gold nanoparticles with an average diameter of approximately 10 to 100 nm are more preferable. Meanwhile, in terms of increasing dispersibility and reducing their unspecific adsorption onto test strips for lateral flow assay, a labeling agent may be stabilized by an appropriate method. For example, when gold nanoparticles are used as labeling agent, they may be stabilized by citric acid, silica or polyethylene glycols.

The second binding component comprising a labeling agent means a binding component having the labeling agent bound to a part of the second binding component through interactions such as covalent bonding, coordination bonding, hydrogen bonding, hydrophobic bonding, and van der Waals force. Such a binding component comprising a labeling agent can be prepared using known chemical methods. If there is a commercially available product that can be used as a binding component comprising a labeling agent, such a commercially available product may be used as well.

<2. Detection Method for detecting an oxLDL/$\beta_2$GPI complex of the present invention>

Below, a detection method for detecting a complex of oxLDL and $\beta_2$-glycoprotein I (an oxLDL/$\beta_2$GPI complex) in a test sample is explained. The term "oxLDL/$\beta_2$GPI complex" is an abbreviation to indicate complex of oxLDL and $\beta_2$-glycoprotein I.

<2.1. Step of capturing an oxLDL/$\beta_2$GPI complex - part 1 - >

In the detection method of the present invention, an oxLDL/$\beta_2$GPI complex in a test sample is captured in a predetermined position, namely detection region 12, on a test strip, by a first binding component that binds to the complex. For example, if the first binding component that binds to an oxLDL/$\beta_2$GPI complex is temporally fixed at detection region 12 on a test strip, an oxLDL/$\beta_2$GPI complex that comes transported thereto is bound to the first binding component that is temporally fixed at detection region 12. Thereby, a step of capturing an oxLDL/$\beta_2$GPI complex in a predetermined position on a test strip by a first binding component is accomplished. As above mentioned, in a preferred embodiment of the detection method of the present invention, a part of the first binding component that binds to an oxLDL/$\beta_2$GPI complex is a first binding member that specifically binds to a $\beta_2$GPI comprised in an oxLDL/$\beta_2$GPI complex. However, the step of capturing an oxLDL/$\beta_2$GPI complex in a predetermined position on a test strip is not limited to the above-mentioned example. For example, the step may be the one as below described.

<2.2. Step of capturing an oxLDL/$\beta_2$GPI complex - part 2 - >

In a preferred embodiment of the detection method of the present invention, the first binding component comprises a first binding unit, which comprises a first specific binding element and the first binding member that specifically binds to a $\beta_2$GPI molecule comprised in an oxLDL/$\beta_2$GPI complex, and a second binding unit, which is placed in the predetermined position on the test strip and comprises a second specific binding element that specifically binds to the first specific binding element. In such a case like this, the step of capturing an oxLDL/$\beta_2$GPI complex in a test sample in a predetermined position on a test strip is performed in two separate steps: i.e., a step of making an oxLDL/$\beta_2$GPI complex and the first binding member comprised in the first binding unit be bound, and a step of making the first specific binding element comprised in the first binding unit and the second specific binding element bound comprised in the second binding unit be bound. It should be noted that the second binding unit comprising the second specific binding element being placed in the predetermined position, namely detection region 12, on a test strip means that the second binding unit is temporally fixed at detection region 12. Herein, any means may be used to temporally fix the second binding unit at detection region 12.

The step of making an oxLDL/$\beta_2$GPI complex and the first binding member comprised in the first binding unit be bound, and the step of making the first specific binding element comprised the first binding unit and the second specific binding element comprised in the second binding unit be bound may be performed simultaneously or separately in any order. However, it is preferable to perform the step of making an oxLDL/$\beta_2$GPI complex and the first binding member comprised in the first binding unit be bound before the step of making the first specific binding element comprised in the first binding unit and the second specific binding element comprised in the second binding unit be bound. The step of making an oxLDL/$\beta_2$GPI complex and the first binding member comprised in the first binding unit be bound may be performed on a test strip, although it may as well be performed outside a test strip. However, it is preferable to perform the step outside a test strip.

For example, the step of making an oxLDL/$\beta_2$GPI complex and the first binding member be bound can be performed outside a test strip and before the step of making the first specific binding element and the second specific binding element be bound, by mixing an oxLDL/$\beta_2$GPI complex and the first binding unit comprising the first binding member and incubating them together in advance so that they are bound, and subsequently using the mixture for lateral flow assay. In such a case like this, it is advantageous in that detection with high sensitivity may be enabled because an oxLDL/$\beta_2$GPI complex and the first binding member can be sufficiently bound in a condition that is appropriate for making the two be bound.

Meanwhile, for example, the step of making an oxLDL/$\beta_2$GPI complex and the first binding member be bound can be performed inside a test strip and before the step of making the first specific binding element and the second specific binding element be bound, by using a test strip with the first binding member coated at a position that is upstream of the predetermined position where the second binding unit is placed (namely, detection region 12) in a manner that the first binding member is diffusible by lateral flow. In such a case like this, the step of making an oxLDL/$\beta_2$GPI complex and the first binding member be bound can be accomplished simply by making a test sample containing an oxLDL/$\beta_2$GPI complex flow through the test strip, without mixing a test sample possibly containing an oxLDL/$\beta_2$GPI complex and the first binding unit and incubating the mixture in advance. Accordingly, the efficiency of detection is advantageously enhanced.

Anyway, when the step of making an oxLDL/$\beta_2$GPI complex and the first binding member be bound is performed before the step of making the first specific binding element comprised in the first binding unit and the second specific binding element comprised in the second binding unit be bound, an oxLDL/$\beta_2$GPI complex comes transported by lateral flow to the predetermined position at which the second binding unit is placed in a state that it is already bound to the first binding unit. The first specific binding element comprised in the first binding unit already bound to an oxLDL/$\beta_2$GPI complex then binds to the second specific binding element comprised in the second binding unit placed in the predetermined position. Thereby, the step of capturing an oxLDL/$\beta_2$GPI complex in the predetermined position is accomplished

<2.3 Specific Binding Element>

In a preferred embodiment of the detection method of the present invention, "specific binding element" comprised in the first binding unit and the second binding unit means a substance or a complex of substances that is able to specifically bind to its counterpart specific binding element. The combination of the first specific binding element and the second specific binding element that may be used in the detection method of the present invention is not limited as long as a pair of specific binding elements can specifically bind to each other. However, examples of a preferable combination may include a combination of substances that can form an avidin-biotin binding pair, a combination of substances that can bind by host-guest interaction, such as crown ether, cyclodextrin and calixarene, and a combination of substances that can bind by antigen-antibody interaction.

In terms of obtaining higher detection sensitivity, it is preferable that dissociation constant of the binding between a first specific binding element and a second specific binding element is smaller. For example, the dissociation constant is preferably less than $10^{-8}$ M, more preferably less than $10^{-10}$ M, and more preferably less than $10^{-12}$ M. Specific examples may include the combination of avidin and biotin, streptavidin and biotin, and neutravidin and biotin. The binding between avidin and biotin, streptavidin and biotin, and neutravidin and biotin is very strong and the dissociation constant is as low as $10^{-15}$ M. By making use of such a strong binding, a complex of an oxLDL/$\beta_2$GPI complex and the first binding unit that comes transported by lateral flow can be efficiently captured without minimal omission in the predetermined position on a test strip. Thereby, a higher detection sensitivity can be achieved. The dissociation constant can be obtained by known experimental method. When using the above raised pairs of substances, either of the pair may be used as either of the first specific binding element or the second specific binding element.

A first binding unit may contain more than two molecules of the first specific binding elements, as long as it does not obstruct the specific binding between the first binding member and a $\beta_2$GPI molecule comprised in an ox-LDL/$\beta_2$GPI complex. In such a case like this, it is advantageous in that a complex of an oxLDL/$\beta_2$GPI complex and the first binding unit that comes transported by lateral flow may be more efficiently captured in the predetermined position on a test strip. Meanwhile, it goes without saying that a second binding unit also may contain more than two molecules of the second specific binding elements.

<2.4 Step of labeling an oxLDL/$\beta_2$GPI complex>

By making the second binding component comprising a labeling agent be bound to the oxLDL/$\beta_2$GPI complex captured in the predetermined position on a test strip, the oxLDL/$\beta_2$GPI complex can be labeled by a detectable labeling agent. In order to label the oxLDL/$\beta_2$GPI complex captured in the predetermined position by a labeling agent, the second binding component comprising the labeling agent may be permeated through membrane 3 of test strip 1 either by dropping a solution containing the second binding component comprising the labeling agent onto sample pad 11 or by immersing a lower edge of sample pad 11 into a solution containing the second binding component, after test samples have passed detection region by lateral flow.

The amount of the labeling agent captured in detection region 12 reflects the amount of oxLDL/$\beta_2$GPI complex captured in detection region 12. Accordingly, oxLDL/$\beta_2$GPI complex in a test sample can be detected or quantified by detecting the presence or absence or the amount of the labeling agent captured in detection region 12

The presence or absence or the amount of labeling agents captured in detection region 12 on test strip 1 can be detected by an appropriate method depending on types of labeling agents used. For example, the reflection light intensity, fluorescence intensity, luminescence intensity, or absorbance at detection region 12 (and at control region 13, if necessary) can be measured, wherein the measurement can be done by using measurement instruments or by taking images of the regions and analyzing the images. OxLDL/$\beta_2$GPI complex in a test sample can be detected or quantified, by comparing measurement data obtained from a test sample containing an unknown concentration of oxLDL/$\beta_2$GPI complex and a standard curve obtained from standard samples containing known concentrations of oxLDL/$\beta_2$GPI complex. As a standard sample, $Cu^{2+}$-oxLDL/$\beta_2$GPI complex may be used, the preparation of which is described later in experimental section.

In terms of obtaining higher detection sensitivity, it is preferable that test strip 1 comprises control region 13, wherein substances capturing the second binding component are immersed or fixed. When test strip 1 is provided with control region 13, the second binding component that is not bound to an oxLDL/$\beta_2$GPI complex at detection region 12 and therefore not captured in detection region 12 would be captured in control region 13. In this case, not only the amount of labeling agent to be captured in detection region 12 increases with the amount of oxLDL/$\beta_2$GPI complex captured in detection region 12, but also the amount of labeling agent to be captured in control region 13 decreases with the amount of oxLDL/$\beta_2$GPI complex captured in detection region 12. By taking a measurement value that reflects the amount of labeling agent captured in control region 13 as well as a measurement value that reflects the amount of labeling agent captured in detection region 12, and by using a ratio of the two measurement values obtained at the two regions (i.e., measurement value obtained at detection region 12/measurement value obtained at control region 13) as measurement data, a higher detection sensitivity can be advantageously achieved.

<3. Detection Kit>

A detection kit of the present invention is a detection kit for detecting an oxLDL/$\beta_2$GPI complex, comprising at least the following (A) to (C);

    (A) a first binding component that binds to an oxLDL/$\beta_2$GPI complex,
    (B) a second binding component comprising a labeling agent, and
    (C) a test strip for lateral flow assay.

In a preferred embodiment, the detection kit of the present invention comprises as the first binding component, a first binding component comprising a first binding member that specifically binds to a $\beta_2$GPI comprised in an ox-

LDL/$\beta_2$GPI complex.

In a preferred embodiment, the detection kit of the present invention comprises as the second binding component, a second binding component comprising a second binding member that binds to an apoB100 comprised in an oxLDL/$\beta_2$GPI complex.

In a further preferred embodiment, the detection kit of the present invention comprises as the first binding component comprising a first binding unit, which comprises a first specific binding element and a first binding member that specifically binds to a $\beta_2$GPI molecule comprised in an oxLDL/$\beta_2$GPI complex, and a second binding unit, which is placed in a predetermined position on a test strip and comprises a second specific binding element that specifically binds to the first specific binding element.

Herein, a first binding component or a second binding member comprised in a first binding component may be provided in a state that they are already temporally fixed in the predetermined position on a test strip for lateral flow assay, or in a state that they are fixed by a user in the predetermined position on a test strip for lateral flow assay. On the other hand, it is preferable that a test strip for lateral flow assay comprised in the detection kit is provided in a state that it is already equipped with sample pad 11 and/or wicking pad 14. However, a test strip may be provided without being equipped with sample pad 11 and/or wicking pad 14, so that a user by himself/herself equips a test strip with sample pad 11 and/or wicking pad 14. The same applies to control region 13.

The detection kit of the present invention can be used in the same manner as explained in <2. Detection method for detecting an oxLDL/$\beta_2$GPI complex of the present inventions Meanwhile, the detection kit of the present invention may include other components as long as it includes the abovementioned (A) to (C). Examples of the other components that may be included in the detection kit of the present invention include but not limited to Cu$^{2+}$-oxLDL/$\beta_2$GPI complex that may be used as a standard sample, a blocking agent, and a diluent used for diluting a test sample.

Further explanation of the present invention is described below, based on experimental examples. However, the present invention is not restricted to the following specific examples.

<Experiment 1. Preparation of anti-$\beta_2$GPI antibody and anti-apoB100 antibody>

An anti-$\beta_2$GPI antibody (hereinafter referred to as "3H3 antibody") and an anti-apoB100 antibody (hereinafter referred to as "2E10 antibody"), used in the present description, were prepared using a known hybridoma method as below described.

As a hybridoma cell producing 3H3 antibody, a 3H3 antibody-producing hybridoma clone 3H3, which was obtained with the method described in Example 3 of Japanese Patent No. 5616592, was used. Meanwhile, as a hybridoma cell producing 2E10 antibody, a mouse-mouse hybridoma N2E10, which was obtained with the method described at paragraph [0020] to [0024] of Japanese Patent Application Publication No. 2005-097203, was used. This mouse-mouse hybridoma N2E10 producing 2E10 antibody was deposited at National Institute of Technology and Evaluation (NITE) International Patent Organism Depositary (IPOD) (1-1-1 Higasi, Tukuba-shi, Ibaraki 305-8566, Japan), received by NITE IPOD on September 2$^{nd}$ 2003, and given the accession number of FERM P-19508.

The 3H3-producing hybridoma cells or 2E10-producing hybridoma cells were cultured in IMDM (Iscove's Modified Dulbecco's Medium) medium supplemented with 2.5%(v/v) fetal bovine serum (FBS), in accordance with the usual procedure. The supernatant of the cell culture medium was collected by centrifugation and subjected to purification by affinity chromatography using a Protein A Sepharose resin. 3H3 antibodies and 2E10 antibodies captured by protein A embedded in the Sepharose resin were eluted with glycine-HCl buffer (pH2.7). After that, the dispersion buffer of the obtained solution was replaced with PBS (Phosphate buffered Saline), in accordance with the usual procedure.

Purities of the obtained 3H3 antibody and 2E10 antibody were verified by SDS-PAGE (sodium-dodecyl sulfate polyacrylamide gel electrophoresis). For SDS-PAGE, polyacrylamide gel with a concentration of 12.5%(w/v) was used and as a reducing agent, 2-mercaptoethanol was used. The result is shown in Figure 2. In Figure 2, samples applied to each lane were molecular weight standard (Lane M), reduced cell culture supernatant (Lane C), effluent obtained during antibody purification (Lane E), and the purified antibodies (Lane P). In Figure 2, lanes P for both 3H3 antibody and 2E10 antibody show strong color developments at positions corresponding to heavy chain (Molecular weight of approximately 50 kDa) and light chain (Molecular weight of approximately 25 kDa) with negligible color developments at other positions. The above result indicates that 3H3 antibody and 2E10 antibody with high purity were obtained.

<Experiment 2. Preparation of biotin-3H3 antibody conjugate>

Next, a biotin-3H3 antibody conjugate used for the following experiments was prepared by conjugating biotin to the 3H3 antibody obtained in above Experiment 1. Specifically, the conjugation was performed using a commercially available biotin labeling kit (Product Name "Biotin Labeling Kit-NH$_2$", Dojindo Laboratories, Japan) according to the manufacturer's manual included in the kit. The procedure is briefly described as follows.

To a solution containing 200 $\mu$g of 3H3 antibody, which was obtained in Experiment 1, "WS Buffer" included in the above biotin-labeling kit was added, so that the final volume of the mixture became 200 μL. After mixing by pipetting, the whole volume of the mixture (200 μL) was added on top of a filtration membrane attached to "Filtration Tube" included in the above biotin-labeling kit. The filtration tube was then subjected to centrifugation at 8,000 g and 4°C for 10 mins. On the other hand, "NH$_2$-Reactive Biotin" included in the above biotin labeling kit was reconstituted in 10 μL of DMSO (Dimethyl Sulfoxide). To the solution remained above the filtration membrane of the filtration tube, 100 μL of "Reaction Buffer" (included in the above biotin labeling kit) was added, followed by the addition of the whole volume of the reconstituted "NH$_2$-Reactive Biotin". The mixture was mixed by pipetting and incubated for 10 mins at 37°C. After the incubation, 100 μL of "WS Buffer" was added to the mixture at the filtration tube, and the mixture was subjected to further centrifugation at 8,000 g and 4°C for 10 mins. In order to remove excess free biotin that was not bound to antibody, 200 μL of "WS Buffer" was added to the solution remained on the filtration membrane of the filtration tube and the filtration tube was subjected to centrifugation at 8,000 g and 4°C for 10 mins. Once more, 200 μL of "WS Buffer" was added to the solution remained on the filtration membrane of the filtration tube and the filtration tube was subjected to centrifugation at 8,000 g and 4°C for 10 mins. Then, the solution remained on the filtration membrane of the filtration tube was added with PBS to give a final volume of 200 μL of a solution containing a biotin-3H3 antibody conjugate. The concentration of the biotin-3H3 antibody conjugate in the obtained solution was determined by BCA protein assay using a commercially available kit (Product Name "Pierce™ BCA Protein Assay Kit", Thermo Fisher Scientific Inc., USA).

<Experiment 3. Preparation of gold nanoparticle-2E10 antibody conjugate>

Next, a gold nanoparticle-2E10 antibody conjugate used for the following experiments was prepared by conjugating a gold nanoparticle with a diameter of 20 nm to the 2E10 antibody obtained in the above Experiment 1. Specifically, the conjugation was conducted using a commercially available gold nanoparticle conjugation kit (Product Name "Naked Gold Conjugation Kit (20nm)", Bioporto Diagnostics A/S, Denmark) according to the manufacturer's manual included in the kit. The procedure is briefly described as follows.

First, the buffer of a suspension containing 2E10 antibody was replaced with ultrapure water from PBS by ultrafiltration. As an ultrafiltration membrane, "Amicon Ultra 0.5 mL Centrifugal Filters 10K" (Merck Millipore, Germany) was used. The suspension containing gold nanoparticles with a diameter of 20 nm, which is included in the above gold nanoparticle conjugation kit, was diluted with ultrapure water so that the optical density (OD) at wavelength of 520 nm was adjusted to be 6 A.U.. Herein, the measurement of the optical density was performed using a spectrophotometer (Product Name "BioSpec-nano", Shimadzu Corporation, Japan). Then, the pH of the diluted suspension containing gold nanoparticles was adjusted to 9.0 by addition of appropriate amount of 0.2 M K$_2$CO$_3$ solution. To the thus prepared suspension containing gold nanoparticles, a suspension containing 2E10 antibody (Concentration of 2E10 antibody: 48 μg/mL) was added and the obtained mixture was incubated for 1 hour. After incubation, "Stabilizing Buffer" included in the above gold nanoparticle conjugation kit was added to the mixture at a volume ratio of the mixture to the stabilizing buffer of 1:5, in order to terminate the conjugation reaction.

<Experiment 4. Preparation of standard samples>

<Experiment 4.1. Isolation of LDL from human serum sample>

LDL (Low-Density Lipoprotein) was isolated from commercially available human serum pool (Cosmo Bio Co., Ltd., Japan) by single spin density-adjusted gradient ultracentrifugation, as below described.

First, three density-adjusted solutions (hereinafter referred to as "DAS") with different density, namely DAS 1 to 3, were prepared. The density of DAS1, DAS2, and DAS3 was 1.006 g/cm$^3$, 1.019 g/cm$^3$, and 1.063 g/cm$^3$, respectively. These DAS 1 to 3 contained 1 mM of EDTA(ethylenediaminetetraacetic acid) and the density was adjusted using KBr (potassium bromide). Next, KBr was added to thawed human serum at a volume-to-weight ratio of 13 mL of human serum to 1.82 g of KBr. After addition of KBr, the thawed human serum and KBr was mixed for 10 mins on ice bath using stirrer. The thus prepared mixture containing human serum (Volume: 23.6 mL), DAS1 (Volume: 18.2 mL), DAS2 (Volume: 18.2 mL), and DAS3 (Volume: 10 mL) were loaded slowly into polycarbonate bottle (Product Name "80PC bottle (C)", Hitachi Koki Co., Ltd.) in an order of DAS3, DAS2, DAS1 and human serum. The polycarbonate bottle loaded with the samples was placed in RP45T angle rotor (Hitachi Koki Co., Ltd.) and subjected to centrifugation at 100,000g and 4°C for 24 hours using SCP85H ultracentrifuge (Hitachi Koki Co., Ltd.). After centrifugation, VLDL (Very Low Density Lipoprotein)-containing fraction and chylomicrons-containing fraction were carefully removed and subsequently LDL-containing fraction was collected. The LDL-containing fraction was concentrated using a commercially available ultrafiltration filter (Product Name "Amicon Ultra 15mL Centrifugal Filters 10K", Merck Millipore, Germany) and the concentrated solution was dialyzed overnight against PBS (pH 7.4). The concentration of LDL in the thus obtained sample after dialysis was measure by BCA protein assay using a commercially available kit (Product Name "Pierce™ BCA Protein Assay Kit", Thermo Fisher Scientific Inc., USA).

<Experiment 4.2. Oxidation of LDL>

The oxidized LDL was prepared by oxidizing the LDL obtained in Experiment 4.1. using copper (II) sulfate (CuSO$_4$) in accordance with the usual procedure. The specific protocol for the oxidation is as described below.

The concentration of a solution containing LDL obtained in Experiment 4.1. was adjusted to have 100 μg/mL of apolipoprotein B (hereinafter also referred to simply as "apoB") after adjustment. To the solution obtained after adjustment, copper (II) sulfate was added so that the concentration of copper (II) sulfate was 5 μM, and thereby the oxidation reaction was initiated. After incubation at 37°C for 16 hours, EDTA (ethylenediaminetetraacetic acid) was added to give a final concentration of EDTA of 1 mM, and thereby the oxidation reaction was terminated. After termination of the oxidation reaction, a part of the resulting solution was taken as a test sample in order to check progress of the oxidation of LDL and was subjected to the later described TBARS (Thiobarbituric Reactive Substance) assay. On the other hand, the remaining solution was purified by overnight dialysis against PBS containing 1 mM EDTA. The purified solution was used for further experiments after appropriate concentration or dilution.

In parallel with the above Experiment 4.2., an LDL sample for a comparative study was also prepared similarly as in the above Experiment 4.2 except that EDTA was added at a concentration of 1 mM when initiating the oxidation reaction. This LDL sample was also incubated at 37°C for 16 hours, and after incubation a part of the solution was taken as a comparative sample, which was used as a negative control in the later described TBARS assay.

<Experiment 4.3 TBARS assay>

Whether LDL was oxidized or not was determined by TBARS assay, which is known in the art. TBARS assay is a method for monitoring the oxidation of lipids, by quantifying the amount of TBARS (thiobarbituric reactive substance) represented by MDA (malondialdehyde) that is produced as byproducts of oxidation reaction of LDL. MDA that is produced as a byproduct of oxidation of unsaturated fatty acid reacts with TBA (thiobarbituric acid) to form a fluorescent MDA-TBA adduct whose amount can be quantified by fluorescence spectrophotometer. The specific protocol of the experiment is as below described.

First, a reaction buffer was prepared by mixing the same amount of 0.67%(w/v) TBA solution and 0.2%(w/v) trichloroacetic acid solution. This reaction buffer was mixed with the test sample or the comparative sample taken in Experiment 4.2., and the mixture was incubated for 30 mins on water bath with a temperature of 100°C. After cooling, the amount of the MDA-TBA adduct, which was produced in the mixture obtained using the test sample and the comparative sample, was quantified using fluorescence spectrophotometer. For fluorescence measurement, the excitation wavelength of 515 nm and the detection wavelength of 553 nm were used.

A standard curve was obtained by performing measurements following the above procedure except that standard samples containing a known amount of MDA (0-60 nmoles) were used instead of the test sample obtained in Experiment 4.2. Based on the thus obtained standard curve, the amount of TBARS comprised in the test sample and the comparative sample prepared in Experiment 4.2. was obtained as MDA equivalent. The result is shown in Figure 3. Figure 3 shows the production of significant amount of MDA-TBA adducts in the test sample, which was prepared by addition of 5 μM of copper (II) sulfate and subsequent incubation for 16 hours at 37°C, indicating progress of the oxidation of LDL in the test sample. In contrast, production of MDA-TBA adducts was negligible in the comparative sample, which was added with 1 mM EDTA when initiating the oxidation reaction in Experiment 4.2., indicating that no oxidation of LDL proceeded in the comparative sample. The above results indicate that Cu$^{2+}$-oxidized LDL was obtained by the oxidation reaction using copper (II) sulfate.

<Experiment 4.4. Complexation of Cu$^{2+}$-oxLDL and β$_2$GPI>

The Cu$^{2+}$-oxLDL prepared in Experiment 4.2. by oxidation of LDL using copper (II) sulfate in accordance with the usual procedure was complexed with β$_2$GPI to form Cu$^{2+}$-oxLDL/β$_2$GPI complex following the below described procedure. Namely, the solution containing an appropriate concentration of Cu$^{2+}$-oxLDL and the solution containing an appropriate concentration of β$_2$GPI were diluted and mixed to obtain a reaction solution with a concentration of Cu$^{2+}$-oxLDL of 100 μg/mL and a concentration of β$_2$GPI of 50 μg/mL. This reaction solution was incubated for 16 hours at 37°C, and thereby Cu$^{2+}$-oxLDL/β$_2$GPI complex was obtained. After the incubation, the resulting reaction solution was cooled to -80°C and made frozen so that the complexation reaction was terminated. The frozen samples were appropriately defrosted when being used in the following experiments. On the other hand, the concentration of Cu$^{2+}$-oxLDL/β$_2$GPI complex in the obtained samples was measured by BCA protein assay using a commercially available kit (Product Name "Pierce™ BCA Protein Assay Kit", Thermo Fisher Scientific, USA).

<Experiment 4.5. Confirmation of formation of $Cu^{2+}$-oxLDL/$\beta_2$GPI complex by electrophoresis>

In order to examine formation of $Cu^{2+}$-oxLDL/$\beta_2$GPI complex, human serum, LDL prepared in Experiment 4.1., $Cu^{2+}$-oxLDL prepared in Experiment 4.2., $\beta_2$GPI, and $Cu^{2+}$-oxLDL/$\beta_2$GPI complex prepared in Experiment 4.4. were subjected to agarose gel electrophoresis. For agarose gel electrophoresis, a commercially available TITAN GEL Universal Plate (Helena Laboratories, USA) was used. Except for the human serum, the loading amount of protein was 8 $\mu$g/mL per well. Meanwhile, the loading amount of human serum was 2 $\mu$L per well. The gel electrophoresis was conducted in barbital electrophoresis buffer (10 mM barbital, 50 mM sodium diethylbarbiturate, 1 mM EDTA, and 15 mM $NaN_3$) at 90V for 30 mins. After electrophoresis, the agarose gel was immersed in fixing solution (60%(v/v) EtOH, 10%(v/v) $CH_3COOH$, 30%(v/v) $H_2O$) for 15 mins and dried in a drying oven for 20 mins at 60°C. After drying, the dried agarose gel was subjected to the staining of protein using Amido black 10B or the staining of lipids using Fat Red7B. Image of the stained agarose gel was shown in Figure 4 (In Figure 4, the result of the protein staining with Amido black 10B was shown in the left side (A), and the result of the lipid staining with Fat Red 7B was shown in the right side (B).). As shown in Figure 4, the migration corresponding to LDL and $Cu^{2+}$-oxLDL was observed in the lanes loaded with LDL and the lanes loaded with $Cu^{2+}$-oxLDL, respectively. In contrast, neither migration of proteins nor migration of lipids was observed in the lanes loaded with $Cu^{2+}$-oxLDL/$\beta_2$GPI complex. This result is consistent with the charge neutralization by binding of $\beta_2$GPI to negatively charged $Cu^{2+}$-oxLDL, indicating the formation of $Cu^{2+}$-oxLDL/$\beta_2$GPI complex in Experiment 4.4.

<Experiment 5. Lateral flow assay>

In order to demonstrate the detection of the amount of oxLDL/$\beta_2$GPI complex in a test sample by the detection method of the present invention, first, an experiment to detect oxLDL/$\beta_2$GPI complex in a standard sample containing a known concentration of oxLDL/$\beta_2$GPI complex was performed. Figure 5 shows the schematic illustration of the procedure of the experiment.

<Experiment 5.1. Test strip>

As a test strip for lateral flow assay, a commercially available test strip (Product Name "gRAD OneDetection-120strips", Bioporto Diagnostics A/S, Denmark) was used.

The test strip has a structure as shown in Figure 1, featuring a sample pad, a detection region, a control region, and a wicking pad in this order from upstream of the flow of a test sample. Biotin-binding proteins are fixed to the detection region. Meanwhile, the mixture of anti-mouse IgG antibody, anti-goat IgG antibody, and anti-rabbit IgG antibody (hereinafter this mixture is referred to as "polyclonal anti-IgG antibody") is fixed to the control region. The detection region and the control region both have a line shape with approximately 1 mm width.

<Experiment 5. 2. Preparation of development solution>

In this experiment, lateral flow assay was conducted using two development solutions: i.e., development solution A containing a test sample and development solution B labeling detection targets. Development solutions A and B were prepared as follows.

<Development solution A>

Development solution A is a development solution containing a test sample and a biotin-3H3 antibody conjugate. In this experiment, development solution A was prepared by mixing a standard sample solution (25 $\mu$L) containing the $Cu^{2+}$-oxLDL/$\beta_2$GPI complex prepared in Experiment 4 at a predetermined concentration (0.1 to 50.0 $\mu$g/mL) and a solution containing the biotin-3H3 conjugate prepared in Experiment 2 (2.5 $\mu$L, Concentration of antibody: 0.1 mg/mL). Herein, the standard sample solution was prepared using the $Cu^{2+}$-oxLDL/$\beta_2$GPI complex prepared in Experiment 4 and a diluting solution (25 $\mu$L, 10 mM borate buffer containing 0.5%(w/v) BSA, pH 7.0).

<Development solution B>

On the other hand, development solution B is a development solution in order to label the oxLDL/$\beta_2$GPI complex, a detection target, and contains gold nanoparticle-2E10 antibody conjugates. Development solution B was prepared by mixing a solution containing the gold nanoparticle-2E10 antibody conjugates prepared in Experiment 3 (2.5 $\mu$L, Concentration of antibody: 48 $\mu$g/mL) and a diluting solution (25 $\mu$L, 10 mM borate buffer containing 0.5%(w/v) BSA, pH 7.0).

The thus obtained development solution A was mixed by pipetting and further incubated at room temperature (25°C) for 15 mins before being used for the below described lateral flow assay. Development solution B was also mixed and incubated in the same manner before being used for the lateral flow assay.

<Experiment 5. 3. Lateral flow assay>

The test strip was held approximately vertically so that the wicking pad heads up while the sample pad heads down. In this state, as shown in Figure 5 over a caption of "Development Solution A", the sample pad was brought into contact with development solution A for 1 min and development solution A was made flow through the test strip. Next, as shown in Figure 5 over a caption of "Development Solution B", the sample pad was brought into contact with developing solution B for 4 mins and development solution B was made to flow through the test strip.

Photograph of test strips after the flow of development solutions A and B is shown in Figure 6. In this experimental example, development solution B contains the gold nanoparticle-2E10 antibody conjugates. The gold nanoparticle-2E10 antibody conjugates bind to the oxLDL/$\beta_2$GPI complex captured in the detection region and the polyclonal anti-IgG antibody fixed to the control region. When gold nanoparticles are bound in the detection region and the control region, visually recognizable (purple to red purple colored) lines appear in the detection region and the control region as shown in Figure 6. Therefore, a person carrying out the experiment can easily confirm success or failure of experiments.

<Experiment 5.4. Detection of labeling agent in the detection region and the control region>

After flow of development solutions A and B, the amount of the labeling agents, in other words, the amount of the gold nanoparticles, captured in the detection region and the controlled region was measured by image analysis using image analysis software (ImageJ). Specific procedure is as shown below. It goes without saying that the other appropriate methods may be used as long as the amount of the labeling agents captured in the detection region and the control region can be obtained.

First, the photograph (Figure 6) of the test strips after flow of development solutions A and B was taken using a camera function of a commercially available smartphone. Herein, the photograph was taken without using flash. The image data of the thus obtained photograph was transferred to a personal computer, and converted to a grayscale image using the image analysis software (ImageJ).

Next, the regions corresponding to the detection region and the control region was selected as ROI (Region of Interest) in the grayscale image. Then, a histogram showing a brightness intensity distribution in the selected ROI was obtained. In the histogram, the x-axis of the histogram represents brightness intensity (gray value), while the y-axis represents the number of pixels having each brightness intensity (gray value) in the selected ROI. Areas under the curve for the histograms corresponding to the detection region and the control region were obtained, and then the ratio of the area under the curve for the histogram corresponding to the detection region to the area under the curve for the histogram corresponding to the control region (i.e., [Area under the curve for the histogram corresponding to the detection region]/[Area under the curve for the histogram corresponding to the control region) was obtained as measurement data.

<Experiment 5.5. Drawing standard curve>

Sets of measurement data obtained in Experiment 5.4. were plotted on a graph with the obtained measurement data (i.e., [Area under the curve for the histogram corresponding to the detection region]/[Area under the curve for the histogram corresponding to the control region]) on the x-axis and the concentration of Cu$^{2+}$-oxLDL/$\beta_2$GPI complex in the standard sample on the y-axis. The result is shown in Figure 7. Figure 7 shows plots corresponding to measurement data together with a standard curve that was obtained based on four parametric logistic (4PL) regression model shown in the following formula 1. As shown in Figure 7, the mean square error (MSE) was 0.002974, R$^2$ value was 0.9809, the sum of the squares of the residuals (SS) was 0.02379, and the standard deviation of the residuals (SYX) was 0.07712 for the standard curve, indicating good reliability of the standard curve. The above result demonstrates that the detection method for detecting an oxLDL/$\beta_2$GPI complex of the present invention is able to be used for detecting and quantifying oxLDL/$\beta_2$GPI complex at least in the concentration of 0.1 to 50.0 $\mu$g/m L.

Formula 1

$$y = d + \frac{a - d}{1 + (\frac{x}{c})^b}$$

<Experiment 6. Comparison with ELISA method>

In order to compare the detection method for detecting an oxLDL/$\beta_2$GPI complex of the present invention with a conventional detection method based on ELISA, an experiment to detect $Cu^{2+}$-oxLDL/$\beta_2$GPI complex in standard samples containing known concentrations of $Cu^{2+}$-oxLDL/$\beta_2$GPI complex using ELISA method was conducted. Specifically, the experiment was conducted as below described in the procedure reported by Kobayashi et al. (Journal of Lipid Research 2003, vol. 44, pp. 716-726) with slight modifications.

To each well of a commercially available microplate for immunoassay (Product Name:"Immulon 2HB", ThermoFisher Scientific, USA), 50 $\mu$L of a solution containing the 3H3 antibody prepared in Experiment 1 (Concentration of antibody: 8$\mu$g/mL, buffer: 10 mM Hepes, 150 mM NaCl, pH7.4) was added, and then the microplate was incubated overnight at 4°C so that the 3H3 antibody got adsorbed. Then, the microplate was blocked with 5%(w/v) Bovine Serum Albumin (BSA). Next, 50 $\mu$L of either Hepes buffer as a reference or standard samples containing known concentrations of the $Cu^{2+}$-oxLDL/$\beta_2$GPI complex prepared in Experiment 4 (Concentration of $Cu^{2+}$-oxLDL/$\beta_2$GPI complex: 0.02 to 2.00 $\mu$g/mL) was added to each well, and then the microplate was incubated for 1 hour at room temperature. After removing liquid from each well, each well was washed with 0.05% Tween-20/PBS mixture. Then, the biotin-2E10 antibody conjugate prepared in Experiment 2 was added to each well. After incubation for 1 hour, the liquid in each well was removed and then each well was washed with 0.05% Tween-20/PBS mixture. Then, a commercially available HRP(Horseradish peroxidase)-streptavidin conjugate was added to each well and incubated for 1 hour. After removing the liquid from each well, each well was washed with 0.05% Tween-20/PBS mixture. Then, TMB (3,3',5,5'-tetramethylbenzidine) and hydrogen peroxide ($H_2O_2$) were added to each well, followed by incubation for 30 mins. After terminating the reaction by addition of 0.3N sulfuric acid, absorbance at a wavelength of 450 nm and absorbance at a wavelength of 650 nm as reference were measured. The ratio of the absorbance at 450 nm to the absorbance at 650 nm was used as measurement data. The results were plotted on a graph with the thus obtained measurement data on the x-axis and the concentration of $Cu^{2+}$-oxLDL/$\beta_2$GPI complex in the standard samples on the y-axis. The result is shown in Figure 8.

Figure 8 shows plots corresponding to measurement data together with a standard curve that was obtained based on four parametric logistic (4PL) regression model shown in the above formula 1. As shown in Figure 8, the mean square error (MSE) was 0.0007411, $R^2$ value was 0.9984, the sum of the squares of the residuals (SS) was 0.01556, and the standard deviation of the residuals (SYX) was 0.03026 for the standard curve, indicating good reliability of the standard curve.

On the other hand, Figure 8 shows that the standard curve obtained using ELISA method reaches plateau at a concentration of $Cu^{2+}$-oxLDL/$\beta_2$GPI complex over 2 $\mu$g/mL. This result indicates that a quantification based on ELISA method loses quantitativeness at a concentration of $Cu^{2+}$-oxLDL/$\beta_2$GPI complex of over 2.0 $\mu$g/mL. Accordingly, when detecting and quantifying oxLDL/$\beta_2$GPI complex using ELISA method, a sample needs to be diluted so that the concentration of oxLDL/$\beta_2$GPI complex in the sample becomes less than 2.0 $\mu$g/mL. However, this additional dilution step involves huge amount of work by a person who performs the test, considering that the concentration of oxLDL/$\beta_2$GPI complex in samples is usually unknown and that single ELISA measurement usually takes as long as approximately 3 hours.

In contrast, as is apparent from Figure 7, the standard curve obtained using the detection method for detecting an oxLDL/$\beta_2$GPI complex of the present invention did not reach plateau at a concentration of 2 $\mu$g/mL and even at a concentration as high as 50.0 $\mu$g/mL. This result indicates that the detection method for detecting an oxLDL/$\beta_2$GPI complex of the present invention would enable detection and quantification of oxLDL/$\beta_2$GPI complex with much broader dynamic range compared to a conventional method based on ELISA.

Meanwhile, the detection method of the present invention also gave quantitative result at relatively low concentrations. Figure 9 shows a graph prepared by plotting on the x-axis the results obtained using ELISA method and on the y-axis the results obtained using the detection method of the present invention, in a relatively low concentration range (0.02 to 1 $\mu$g/mL) where ELISA method is very quantitative. As shown in Figure 9, the measurement data obtained using the detection method of the present invention showed very good correlation with the measurement data obtained using ELISA method ($R^2$=0.9871). This result indicates that the detection method of the present invention would enable detection and quantification of oxLDL/$\beta_2$GPI complex as quantitative as ELISA method even in a low concentration range (0.02 to 1 $\mu$g/mL) where ELISA method is very quantitative.

<Experiment 7. Detection of an oxLDL/$\beta_2$GPI complex in serum samples>

Using the detection method for detecting an oxLDL/$\beta_2$GPI complex of the present invention, detection of an oxLDL/$\beta_2$GPI complex in serum samples contained at an unknown concentration was attempted. Specifically, detection of an oxLDL/$\beta_2$GPI complex was carried out similarly as in Experiment 5 except that in total 9 samples of human sera (samples A to I) were used instead of the standard samples when preparing development solution A.

In this experiment, a standard curve was obtained similarly as in Experiment 5, by using standard samples containing known concentrations (0.1 to 50.0 $\mu$g/mL) of the $Cu^{2+}$-oxLDL/$\beta_2$GPI complex prepared in Experiment 4. The thus obtained standard curve is shown in Figure 10. As shown in Figure 10, the mean square error (MSE) was 0.01454, $R^2$ value was 0.959, the sum of the squares of the residuals (SS) was 0.1308, and the standard deviation of the residuals (SYX) was 0.1618 for the standard curve, indicating good reliability of the standard curve. In this experiment, the amounts of oxLDL/$\beta_2$GPI complex in the serum samples were determined semi-quantitatively by fitting the measurement data obtained from the serum samples containing unknown concentrations of oxLDL/$\beta_2$GPI complex to the standard curve. The results for the nine human serum samples (samples A to I) obtained with the detection method of the present invention were shown in Figure 11.

Meanwhile, as a comparison in order to examine the validity of the obtained results, detection of an oxLDL/$\beta_2$GPI complex in the same nine human serum samples was also carried out using ELISA method.

Herein, for ELISA method as well, a standard curve was obtained similarly as in Experiment 6, by using standard samples containing known concentrations (0.02 to 20 $\mu$g/mL) of the $Cu^{2+}$-oxLDL/$\beta_2$GPI complex prepared in Experiment 4. The thus obtained standard curve is shown in Figure 12. As shown in Figure 12, the mean square error (MSE) was 0.0007411, $R^2$ value was 0.9984, the sum of the squares of the residuals (SS) was 0.01556, and the standard deviation of the residuals (SYX) was 0.03026 for the standard curve, indicating good reliability of the standard curve. In this experiment, the amounts of oxLDL/$\beta_2$GPI complex in the serum samples were obtained semi-quantitatively by fitting the measurement data obtained from the serum samples containing unknown concentrations of oxLDL/$\beta_2$GPI complex to the standard curve. The results for the nine human serum samples (samples A to I) obtained with an ELISA method were shown in Figure 13.

Furthermore, Figure 14 shows a graph prepared by plotting the results shown in Figure 11 obtained by the detection method of the present invention on the y-axis and the result shown in Figure 13 obtained by an ELISA method on the x-axis. As shown in Figure 14, the results from the two methods show very good correlation ($R^2$=0.6399). This result indicates that the detection method for detecting an oxLDL/$\beta_2$GPI complex of the present invention would enable effective and reliable detection of an oxLDL/$\beta_2$GPI complex in serum or a variety of samples including serum.

<Experiment 8. Another example of the detection method of the present invention>

An experiment to detect oxLDL/$\beta_2$GPI complex in standard samples containing known concentrations of $Cu^{2+}$-oxLDL/$\beta_2$GPI conjugates was conducted similarly as in Experiment 5 except that development solution A containing biotin-2E10 antibody conjugates instead of the biotin-3H3 antibody conjugates and development solution B containing gold nanoparticle-3H3 antibody conjugates instead of the gold nanoparticle-2E10 antibody conjugates were used. Herein, the biotin-2E10 antibody conjugate was prepared following a procedure similar to the one described in Experiment 2 except that 2E10 antibody was used instead of 3H3 antibody. Meanwhile, the gold nanoparticle-3H3 antibody conjugate was prepared following a procedure similar to the one described in Experiment 3 except that 3H3 antibody was used instead of 2E10 antibody.

Sets of obtained data were plotted on a graph with the obtained measurement data (i.e., [Area under the curve for the histogram corresponding to the detection region]/[Area under the curve for the histogram corresponding to the control region) on the x-axis and the concentration of $Cu^{2+}$-oxLDL/$\beta_2$GPI complex in the standard samples on the y-axis. The result is shown in Figure 15. Figure 15 shows plots corresponding to measurement data together with a standard curve that was obtained based on four parametric logistic (4PL) regression model shown in the above formula 1. As shown in Figure 15, the mean square error (MSE) was 0.00006322, $R^2$ value was 0.9984, the sum of the squares of the residuals (SS) was 0.0005058, and the standard deviation of the residuals (SYX) was 0.01124 for the standard curve, indicating good reliability of the standard curve. Meanwhile, the above result also indicates that the detection method of present invention would enable detection and quantification of oxLDL/$\beta_2$GPI complex even when anti-apoB100 antibody that binds to an apoB100 comprised in an oxLDL/$\beta_2$GPI complex is used as a substance to provide the first binding member comprised in the first binding component, and anti-$\beta_2$GPI antibody that binds to a $\beta_2$GPI comprised in an oxLDL/$\beta_2$GPI complex is used as a substance to provide the second binding member comprised in the second binding component.

Nevertheless, when comparing the standard curve shown in Figure 15 obtained in Experiment 8 and the standard curve shown in Figure 7 obtained in the afore-mentioned Experiment 5, the detection method in Experiment 5 gave greater signal and had better sensitivity compared to the detection method in Experiment 8. This result indicates that the detection method of the present invention has an outstanding sensitivity when anti-$\beta_2$GPI antibody that binds to a $\beta_2$GPI comprised in an oxLDL/$\beta_2$GPI complex is used as a substance to provide the first binding member comprised in the first binding component, and anti-apoB100 antibody that binds to an apoB100 comprised in an oxLDL/$\beta_2$GPI complex is used as a substance to provide the second binding member comprised in the second binding component.

Industrial Applicability

The detection method for detecting an oxLDL/$\beta_2$GPI complex and the detection kit of the present invention would enable quick and easy detection and quantification with a broad dynamic range of oxLDL/$\beta_2$GPI complex comprised in a test sample including biological samples. The application to high-throughput detection and diagnosis of arteriosclerotic diseases would be expected. Thus, the industrial applicability of the present invention is huge.

Explanation of Symbols

1    Test Strip for Lateral Flow Assay
2    Supporting member
3    Membrane
11    Sample Pad
12    Detection Region
13    Control Region
14    Wicking Pad

Claims

1.  A detection method for detecting a complex of oxidized LDL and $\beta_2$-glycoprotein I (an oxLDL/$\beta_2$GPI complex) in a

test sample, which uses a test strip for lateral flow assay, comprising;

a step of capturing the oxLDL/$\beta_2$GPI complex in the test sample in a predetermined position on the test strip by a first binding component that binds to the oxLDL/$\beta_2$GPI complex; and
a step of labeling the oxLDL/$\beta_2$GPI complex captured in the predetermined position on the test strip by making a second binding component comprising a labeling agent be bound to the captured oxLDL/$\beta_2$GPI complex.

2. The method as claimed in claim 1, wherein the first binding component comprises a first binding member that specifically binds to a $\beta_2$GPI molecule comprised in the oxLDL/$\beta_2$GPI complex.

3. The method as claimed in claim 2,

wherein the first binding component comprises a first binding unit, which comprises a first specific binding element and the first binding member that specifically binds to the $\beta_2$GPI molecule comprised in the oxLDL/$\beta_2$GPI complex, and a second binding unit, which is placed in the predetermined position on the test strip and comprises a second specific binding element that specifically binds to the first specific binding element, and
wherein the step of capturing the oxLDL/$\beta_2$GPI complex in the test sample in the predetermined position on the test strip comprises, a step of making the oxLDL/$\beta_2$GPI complex and the first binding member comprised in the first binding unit be bound, and a step of making the first specific binding element of the first binding unit and the second specific binding element of the second binding unit be bound.

4. The method as claimed in claim 3,
wherein the first specific binding element and the second specific binding element are either avidin and biotin or *vice versa,* streptavidin and biotin or *vice versa,* or neutravidin and biotin or *vice versa,* respectively.

5. The method as claimed in any one of claims 2 to 4, wherein binding between the first binding member and the oxLDL/$\beta_2$GPI complex is based on antigen-antibody binding.

6. The method as claimed in any one of claims 1 to 5, wherein the second binding component comprises a second binding member that specifically binds to an apolipoprotein B-100 (apoB100) comprised in the oxLDL/$\beta_2$GPI complex.

7. The method as claimed in claim 6, wherein binding between the second binding member and the oxLDL/$\beta_2$GPI complex is based on antigen-antibody binding.

8. A detection kit for carrying out the method as claimed in any one of claims 1 to 7, which at least comprises the following (A) to (C):

(A) a first binding component that binds to an oxLDL/$\beta$2GPI complex;
(B) a second binding component comprising a labeling agent; and
(C) a test strip for lateral flow assay.

Figure 1

Figure 2

Figure 3

(A) Amido Black 10B          (B) Fat Red 7B

Figure 4

Immersion for 1 min — Immersion for 4 mins — Take photograph — Image Analysis

Development Solution A
- Biotin-3H3 antibody (anti-β₂GPI antibody) conjugate
- oxLDL/β₂GPI complex-containing sample

Development Solution B
- Gold nanoparticle-2E10 antibody (anti-apoB100 antibody) conjugate

Figure 5

Concentration of Cu²⁺-oxLDL/ β₂GPI complex in standard samples

0 µg/mL
0.10 µg/mL
0.25 µg/mL
0.50 µg/mL
1.00 µg/mL
2.50 µg/mL
5.00 µg/mL
10.00 µg/mL
25.00 µg/mL
50.00 µg/mL

Control Region    Detection Region

Figure 6

| a | 0.0379 |
|---|---|
| b | 0.7981 |
| c | 3.328 |
| d | 1.491 |
| MSE | 0.002974 |
| R² | 0.9809 |
| SS | 0.02379 |
| SY X | 0.07712 |

Figure 7

| a | 0.07178 |
|---|---|
| b | 1.311 |
| c | 0.3629 |
| d | 2.139 |
| MSE | 0.0007411 |
| R² | 0.9984 |
| SS | 0.01556 |
| SY X | 0.03026 |

Figure 8

Figure 9

Figure 10

| Serum Sample | oxLDL/β₂GPI complex concentration(μg/mL) |
|:---:|:---:|
| A | 0.055 |
| B | 0.122 |
| C | 0.031 |
| D | 0.014 |
| E | 0.036 |
| F | 0.176 |
| G | 0.323 |
| H | 0.031 |
| I | 0.148 |

Figure 11

Figure 12

| Serum Sample | oxLDL/$\beta_2$GPI complex concentration($\mu$g/mL) |
|---|---|
| A | 0.257 |
| B | 0.078 |
| C | 0.090 |
| D | 0.011 |
| E | 0.108 |
| F | 0.224 |
| G | 0.406 |
| H | 0.129 |
| I | 0.328 |

Figure 13

$$y = 0.6188x - 0.0081$$
$$R^2 = 0.6399$$

Concentration of oxLDL/$\beta_2$GPI complex ($\mu$g/mL) determined by the detection method of the present invention

Concentration of oxLDL/$\beta_2$GPI complex ($\mu$g/mL) determined by ELISA method

Figure 14

Figure 15

## INTERNATIONAL SEARCH REPORT

| International application No. |
| --- |
| PCT/JP2021/003773 |

A. CLASSIFICATION OF SUBJECT MATTER
G01N 33/53(2006.01)i; G01N 33/543(2006.01)i
FI: G01N33/53 W; G01N33/53 V; G01N33/543 521
According to International Patent Classification (IPC) or to both national classification and IPC

B. FIELDS SEARCHED

Minimum documentation searched (classification system followed by classification symbols)
G01N33/53; G01N33/543

Documentation searched other than minimum documentation to the extent that such documents are included in the fields searched
Published examined utility model applications of Japan       1922–1996
Published unexamined utility model applications of Japan     1971–2021
Registered utility model specifications of Japan             1996–2021
Published registered utility model applications of Japan     1994–2021

Electronic data base consulted during the international search (name of data base and, where practicable, search terms used)
JSTPlus/JMEDPlus/JST7580 (JDreamIII)

C. DOCUMENTS CONSIDERED TO BE RELEVANT

| Category* | Citation of document, with indication, where appropriate, of the relevant passages | Relevant to claim No. |
| --- | --- | --- |
| A | JP 2008-8894 A (OKAYAMA UNIVERSITY) 17 January 2008 (2008-01-17) entire text, all drawings | 1-8 |
| A | WO 03/022866 A1 (MATSUURA, Eiji) 20 March 2003 (2003-03-20) entire text, all drawings | 1-8 |
| A | WO 95/09363 A1 (YAMASA CORPORATION) 06 April 1995 (1995-04-06) entire text, all drawings | 1-8 |
| A | JP 2003-227837 A (IKAGAKU CO., LTD.) 15 August 2003 (2003-08-15) entire text, all drawings | 1-8 |
| A | JP 2002-17353 A (JAPAN TOBACCO INC.) 22 January 2002 (2002-01-22) entire text, all drawings | 1-8 |
| A | WO 2009/154026 A1 (OKAYAMA UNIVERSITY) 23 December 2009 (2009-12-23) entire text, all drawings | 1-8 |

☒ Further documents are listed in the continuation of Box C.    ☒ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 24 March 2021 (24.03.2021) | 06 April 2021 (06.04.2021) |

| Name and mailing address of the ISA/ | Authorized officer |
| --- | --- |
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**

International application No.

PCT/JP2021/003773

C (Continuation).  DOCUMENTS CONSIDERED TO BE RELEVANT

| Category* | Citation of document, with indication, where appropriate, of the relevant passages | Relevant to claim No. |
|---|---|---|
| A | KOBAYASHI, kazuo et al., "A specific ligand for β 2-glycoprotein I mediates autoantibody-dependent uptake of oxidized low density lipoprotein by macrophages", Journal of Lipid Research, 2001, vol. 42, pp. 697-709, entire text, all drawings | 1-8 |

Form PCT/ISA/210 (continuation of second sheet) (January 2015)

**INTERNATIONAL SEARCH REPORT**
Information on patent family members

International application No.

PCT/JP2021/003773

| Patent Documents referred in the Report | Publication Date | Patent Family | Publication Date |
|---|---|---|---|
| JP 2008-8894 A | 17 Jan. 2008 | US 2010/0081149 A1 entire text, all drawings | |
| WO 03/022866 A1 | 20 Mar. 2003 | US 2004/0241858 A1 entire text, all drawings | |
| WO 95/09363 A1 | 06 Apr. 1995 | US 5900359 A entire text, all drawings EP 722086 A1 | |
| JP 2003-227837 A | 15 Aug. 2003 | US 2003/0077668 A1 entire text, all drawings EP 1070962 A2 | |
| JP 2002-17353 A | 22 Jan. 2002 | EP 1046652 A1 entire text, all drawings | |
| WO 2009/154026 A1 | 23 Dec. 2009 | US 2011/0182817 A1 entire text, all drawings | |

Form PCT/ISA/210 (patent family annex) (January 2015)

32

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5616592 B

- JP 2005097203 A

**Non-patent literature cited in the description**

- *Journal of Lipid Research,* 2001, vol. 42 (5), 697-709
- *Journal of Lipid Research,* 2001, vol. 42 (9), 1346-1367

- *Biological and Pharmaceutical Bulletin,* 2016, vol. 39 (1), 1-24
- **KOBAYASHI et al.** *Journal of Lipid Research,* 2003, vol. 44, 716-726